# EUROPEAN PATENT APPLICATION

(11) **EP 0 591 544 A1**
(43) Date of publication of application: **13.04.1994**
(21) Application number: 93906822.7
(22) Date of filing: 25.03.1993
(51) Int. Cl.: A61K 49/00

(54) **REAGENT FOR NUCLEAR MAGNETIC RESONANCE**

(30) Priority: 26.03.1992 JP 67920/92
(71) Applicant: ARAI, Toshiyuki, Kyoto-shi, Kyoto 606 (JP); MORI, Kenjiro, Sakyo-ku, Kyoto-shi, Kyoto 606 (JP)
(72) Inventor: ARAI, Toshiyuki, Kyoto-shi, Kyoto 606 (JP); MORI, Kenjiro, Sakyo-ku, Kyoto-shi, Kyoto 606 (JP)
(74) Representative: Stachow, E.-W., Prof. Dr.
(86) International application number: JP9300358
(87) International publication number: WO9318796

(57) **Abstract**

A reagent for nuclear magnetic resonance composed of a first inhalation gas containing oxygen at least in a concentration necessary for life supporting and a second inhalation gas to be supplied in the same amount as that of the first gas after the first gas is supplied for a given time. The second inhalation gas contains oxygen in the same concentration as that of the oxygen contained in the first gas and the concentration of the oxygen isotope ¹⁷O contained in the second gas is higher than that of the oxygen isotope ¹⁷O contained in the first gas, so that the blood oxyhemoglobin level after the supply of the first inhalation gas can be kept constant even after the supply of the second inhalation gas. Therefore, a change in the signal detected by NMR represents a change caused solely by the production of H₂¹⁷O without being affected by a change in the oxyhemoglobin level, thus providing information regarding metabolic functions.

## Description

### (Technical Field)

The present invention concerns a nuclear magnetic resonance agent which is used upon causing hydrogen H in H₂¹⁶O present in a living body to nuclear magnetic resonance and detecting change with time of resonance signals thereof.

### (Background Art)

Nuclear magnetic resonance tomography (NMR-CT) used for a nuclear magnetic resonance diagnostic method can conduct tomograph for an inclined surface at an optional angle more easily as compared with conventional X-ray CT scanners, further does not suffer from hindrance for imaging by bones or air and has extremely high safety with no worry of causing X-ray exposure.

Then, tomographic images are formed, for instance, by irradiating sinusoidal signals depending on the resonance frequency of hydrogen atom H to a living body and detecting the state of the proton of hydrogen H present as a water content H₂O in the living body by detecting resonance signals.

By the way, oxygen includes, in addition to ordinary oxygen ¹⁶O, radioisotope ¹⁵O and stable isotope ¹⁷O, in which the stable isotope ¹⁷O has quite the same chemical property as that of ordinary oxygen ¹⁶O and, accordingly, it gives no undesired effects at all when it is intaken into the living body and is served to the metabolism in the same manner as ordinary oxygen.

Further, in the tomography by NMR-CT, since the relaxation time is different between a resonance signal issued from a water content present as H₂¹⁷O and a resonance signal issued from a water content present as H₂¹⁶O, if the intensity of the resonance signal that changes by administration of ¹⁷O into the living body, information about the metabolism can be obtained.

In view of the above, the present inventor has proposed a nuclear magnetic resonance agent incorporated with ¹⁷O at a presence ratio greater than the naturally existing ratio (refer to Japanese Patent Laid-Open Hei 3-167843).

According to this, since H₂¹⁷O is produced and increased by the metabolism of living cells merely by inhalating the nuclear magnetic resonance agent, if NMR-CT detects, for example, the proton of H₂¹⁶O, since the relaxation time for the proton is shortened for a portion in which the metabolism is vigorous to produce a great amount of H₂¹⁷O, the signal intensity is weakened, whereas the signal intensity does not change for a portion in which the metabolism is failed to produce no H₂¹⁷O such as a focal portion.

Accordingly, it can be judged simply where the focal portion is present by detecting the change of the signal intensity.

However, a subsequent study has revealed that the change of the signal intensity and the state of the metabolism do not always agree.

For instance, if an oxygen gas containing a great amount of ¹⁷O is inhaled, since a great amount of H₂¹⁷O is produced at least in a healthy portion, the relaxation time of the proton must be shortened to weaken the signal intensity, but it has actually been found for such an example as the signal intensity does not change or, on the contrary, the signal intensity is increased.

Then, as a result of a further study, it has been found that the signal intensity suffers from the concentration of oxyhemoglobin in blood and the signal intensity is increased more as the concentration of oxyhemoglobin in blood goes higher, irrespective of the fact whether oxygen bonded to oxyhemoglobin is ¹⁶O or ¹⁷O.

In view of the above, it is a technical subject of the present invention to maintain the concentration of oxyhemoglobin in blood constant and detect the change with time of the signal intensity due to the production of H₂¹⁷O, thereby enabling to accurately recognize information regarding the metabolic function based on the above finding.

### (Disclosure of the Invention)

In order to overcome the subject, the present invention has a feature in a nuclear magnetic resonance agent which is used upon causing proton H of H₂¹⁶O present in a living body to nuclear magnetic resonance and detecting the change with time of the resonance signal thereof comprising a first inhalation gas incorporated with oxygen at a concentration required for sustaining life and a second inhalation gas to be supplied at an identical amount of supply with the first inhalation gas after the supply of the first inhalation gas for a predetermined period of time, in which the second inhalation gas is incorporated with oxygen at an identical concentration with the oxygen concentration in the first inhalation gas, and an oxygen isotope ¹⁷O is contained in the oxygen at a concentration higher than the concentration of the oxygen isotope ¹⁷O contained in the first inhalation gas.

With this constitution, when the first inhalation gas is at first supplied for a predetermined period of time and, subsequently, the second inhalation gas is supplied at an identical amount of supply with the first inhalation gas, since the second inhalation gas is incorporated with oxygen at an identical concentration with the oxygen concentration in the first inhalation gas, the concentration of oxyhemoglobin in blood is maintained at a constant value between a case of supplying a first inhalation gas and a case of supplying the second inhalation gas.

Further, since the second inhalation gas is incorporated with the oxygen isotope ¹⁷O at a predetermined concentration higher than the concentration of the oxygen isotope ¹⁷O contained in the first inhalation gas, ¹⁷O is carried by blood to cells and H₂¹⁷O is produced by the metabolic function of the cells.

Accordingly, signals detected by NMR do not suffer from the effect of the change of the concentration of oxyhemoglobin and show the change caused by the production of H₂¹⁷O, to obtain information regarding the metabolic function.

For instance, if the metabolic function is normal, since the relaxation time of the proton is shortened when the second inhalation gas is supplied, as compared with the case of inhalating the first inhalation gas, the signal intensity is surely lowered. Further, if the metabolic function is abnormal, since the amount of H₂¹⁷O newly produced is small, the reduction rate of the signal intensity is small. If the metabolic function is failed, the signal intensity does not change since H₂¹⁷O is not produced.

Furthermore, when a first inhalation gas incorporated with oxygen isotope ¹⁷O in the oxygen at a concentration lower than the naturally existing ratio is used, change of the signal intensity appears remarkably.

### (Brief Explanation of the Drawings)

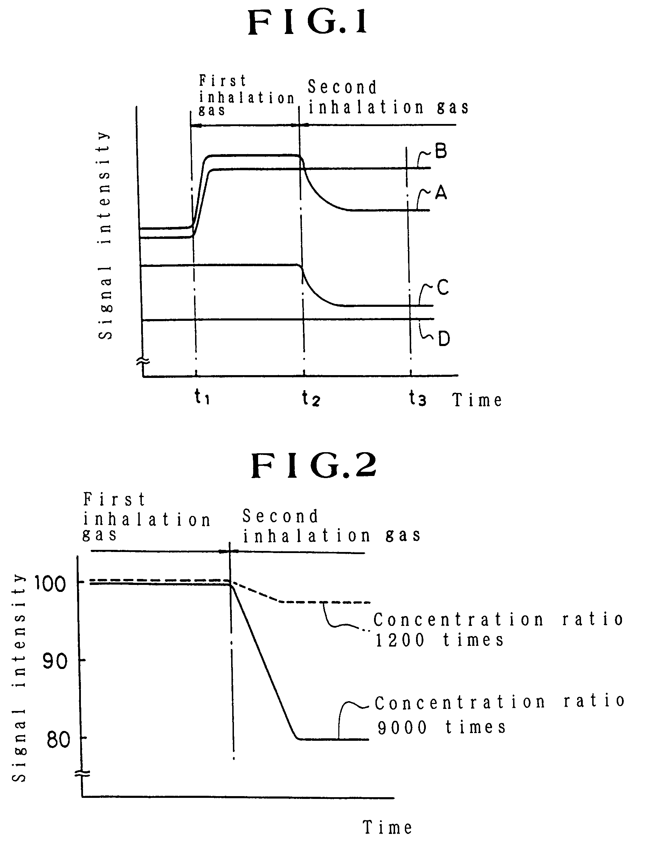

Fig. 1 is a graph illustrating the change of the signal intensity detected by using the nuclear magnetic resonance agent according to the present invention and Fig. 2 is a graph illustrating difference of the change of the signal intensity by the nuclear magnetic resonance agent.

### (Best Mode Practicing the Invention)

The present invention will hereinafter be explained with reference to concrete examples.

The nuclear magnetic resonance agent according to the present invention comprises a first inhalation gas incorporated with oxygen at a predetermined concentration higher than a concentration required for sustaining life and a second inhalation gas incorporated with oxygen at an identical concentration with the oxygen concentration in the first inhalation gas, in which the oxygen isotope ¹⁷O in the oxygen is contained at a predetermined concentration higher than the concentration of the oxygen isotope ¹⁷O contained in the first inhalation gas.

As the first inhalation gas any of non-toxic gases incorporated with oxygen required for sustaining life may be used, for example, 40% of oxygen gas and 60% of nitrogen gas in which oxygen isotope ¹⁷O is contained in oxygen at 0.037% (naturally existing ratio) and the concentration of oxygen is selected to higher than that the usual air.

Further, in the second inhalation gas, the oxygen concentration is identical with the first inhalation gas, in which 40% of oxygen gas and 60% of nitrogen gas are contained and an oxygen isotope ¹⁷O is contained in the oxygen at a ratio higher than that in the first inhalation gas (for example, 40%).

It may suffice that the concentration of oxygen is identical to such an extent as giving no effects on signals detected by NMR.

Then, when the first inhalation gas is supplied to a living body, oxygen contained in the gas is intaken by inhalation into a lung. Since the amount of the oxygen isotope ¹⁷O contained in the oxygen of the first inhalation gas is very small, ¹⁶O is bonded with hemoglobin in blood to form oxyhemoglobin, which is circulated through a blood flow in a body and H₂¹⁶O is produced by the metabolic function of the cells.

Then, inhalation is continued for a predetermined period of time and tomography is conducted by detecting the proton of ¹⁶O by NMR-CT till the concentration of oxyhemoglobin in blood is stabilized with lapse of time before and after the inhalation to take images for the structure of a living tissue or record the signal intensity for a specified portion.

For instance, Fig. 1 is a graph illustrating the change of the signal intensity detected by using the nuclear magnetic resonance agent according to the present invention in which the ordinate indicates signal intensity and the abscissa indicates time.

In the drawing, A is a signal intensity for a portion in which both of the blood flow and the metabolic function are normal, B is a signal intensity for a portion in which the blood flow is normal but the metabolic function is abnormal, C is a signal intensity for a portion in which the metabolic function is normal but the blood flow is abnormal and D is a signal intensity for a portion in which both of the blood flow and the metabolic function are abnormal.

At first, when the first inhalation gas is supplied continuously for a predetermined period of time, since the first inhalation gas is incorporated with oxygen at a concentration higher than usual air, the concentration of oxyhemoglobin in blood is increased. When the change of the signal intensity in this course is detected (Fig. 1, t₁ - t₂), since a great amount of oxyhemoglobin is supplied to a portion where the blood fluid is normal, the signal intensity is increased (refer to Fig. 1, A, B), whereas since oxyhemoglobin is not supplied in a great amount in a case where the blood flow is abnormal, the signal intensity is low (refer to Fig. 1, C, D).

Accordingly, information about the distribution of the blood flow can be obtained from the signal intensity.

Then, when the supply of the first inhalation gas is interrupted and, at the same time, supply of the second inhalation gas is started at an identical amount of supply with that of the first inhalation gas (Fig. 1 t₂), since the concentration of oxygen in the second inhalation gas is identical with the first inhalation gas, the concentration of oxyhemoglobin in blood does not change by the supply of the second inhalation gas, and the total amount of water produced by the metabolic function is also identical with that in a case of supplying the first inhalation.

Then, since the second inhalation gas is incorporated with ¹⁷O at a concentration higher than that in the first inhalation gas, the signal intensity for a portion where the cell metabolic function is normal (refer to Fig. 1, A, B) is gradually lowered since the relaxation time of proton is shortened due to the production of H₂¹⁷O) (Fig. 1, t₂ - t₃), the portion becomes dark in tomographic images, and the intensity of the signals is stabilized with lapse of a predetermined period of time (Fig. 1 t₃).

Since the reduction of the signal intensity depends only on the change of the amount of H₁¹⁷O produced, the signal for a portion where the metabolic function is vigorous and a great amount of H₂¹⁷O is produced shows a great variation coefficient and lowers abruptly, whereas the signal for a portion where the metabolic function becomes weak and the amount of H₂¹⁷O produced is small shows small variation coefficient and lowers moderately.

On the other hand, in a portion where the metabolic function is failed since H₂¹⁷O is not produced even if the second inhalation gas is supplied, the signal intensity (refer to Fig. 1, B, D) does not change (Fig. t₂ - t₃).

As described above, since the concentration of oxygen is equal between the first inhalation gas and the second inhalation gas, the concentration of oxyhemoglobin in blood is maintained content even when the second inhalation gas is supplied and the change of the signal intensity is caused only by the production of H₂¹⁷O. It can be judged that the metabolic function is vigorous if the signal intensity lowers abruptly and can be judged that the metabolic function is deteriorated if the signal intensity lowers moderately, and it can be judged that the metabolic function is failed in a case where the signal intensity does not change.

Further, the composition for each of the magnetic resonance agents is not restricted to that in the example and it is only necessary that the first inhalation gas comprises, like that usual air, about 21% of oxygen and about 78% of nitrogen in which the oxygen isotope ¹⁷O is contained in the oxygen at a naturally existing ratio of 0.037%, while the second inhalation gas contains about 21% of oxygen and about 78% of nitrogen, in which the oxygen isotope ¹⁷O is contained in the oxygen at a concentration higher than the naturally existing ratio of 0.037% (for example, 45%).

However, in the case of such a composition, since the concentration of oxyhemoglobin in blood does not increase, information as far as the distribution of the blood can not be recognized as in the case of the example but only the information about the metabolic function can be recognized. That is, when it is intended to obtain both of the information for the blood flow and the metabolism, the oxygen concentration of the first inhalation gas has to be higher than the oxygen concentration in usual air.

Further, in a case of using the first inhalation gas incorporated with oxygen isotope ¹⁷O at a naturally existing ratio and a second inhalation gas incorporated with 45% of the oxygen isotope as described above, when the second inhalation gas is inhaled after inhalation of the first inhalation gas, although the signal intensity does surely change to cause a difference of brightness and darkness upon taking images but the change can not sometime be discriminated clearly because of a technical reason such as the sensitivity of sensors.

In view of the above, when the first inhalation gas incorporated with the oxygen isotope ¹⁷O at a concentration lower than the naturally existing ratio (for example, 0.005%) and the second inhalation gas incorporated with the oxygen isotope ¹⁷O at a concentration higher than the naturally existing ratio (for example, 45%) are used, the concentration ratio of the oxygen isotope between the first inhalation gas and the second inhalation gas is only about 1200 times in the previous example but the concentration reaches as large as about 9000 times in this example.

Fig. 2 is a graph illustrating a difference of the signal change in a case where concentration ratio is different and it can be seen that the signal intensity lowers only by about 3% or less in a case of a concentration ratio at 1200 times (illustrated by a dotted chain), whereas it lowers as great as 20% in a case at a concentration ratio of 9000 times (illustrated by solid line).

Since the change of the signal intensity of about 3% may some time be caused when the measuring conditions vary by some or other external factors, it is difficult to judge whether the signal change is caused by the production of H₂¹⁷O or not. On the other hand, since the signal intensity change as large as 20% is not caused by the change of the measuring conditions, it can be judged that a portion is normal due to the production of H₂¹⁷O.

Further, if the signal intensity changes as great as by 20%, it can be judged simply whether other portion is a normal or not by detecting the signal change in other portion at that instance.

Further, when a usual nuclear magnetic resonance apparatus is used for taking images, the signal change for a normal portion appears extremely distinct as the change of brightness and darkness and, if no brightness - darkness change is caused for other portion when such brightness - darkness change is caused, it can be judged there is a certain abnormality in the portion.

### (Industrial Applicability)

As has been described above according to the present invention, since the concentration of oxyhemoglobin in blood does not change between a case of supplying the first inhalation gas and a case of supplying the second inhalation gas, the change of the signal intensity is caused by the amount of H₂¹⁷O produced by the metabolic function, and information on the metabolic function can be recognized accurately by detecting change with time of the signal intensity.

## Claims

1. A nuclear magnetic resonance agent which is used upon causing proton H of H₂¹⁶O present in a living body to nuclear magnetic resonance and detecting the change with time of the resonance signal thereof comprising a first inhalation gas incorporated with oxygen at a concentration required for sustaining life and a second inhalation gas to be supplied at an identical amount of supply with the first inhalation gas after the supply of the first inhalation gas for a predetermined period of time, in which the second inhalation gas is incorporated with oxygen at an identical concentration with the oxygen concentration in the first inhalation gas, and an oxygen isotope ¹⁷O is contained in the oxygen at a concentration higher than the concentration of the oxygen isotope ¹⁷O contained in the first inhalation gas.

2. A nuclear magnetic resonance agent as defined in claim 1, wherein oxygen isotope ¹⁷O is incorporated in the oxygen of the first inhalation gas at a concentration lower than the naturally existing ratio and the oxygen isotope ¹⁷O is incorporated in the oxygen of the second inhalation gas at a concentration higher than the naturally existing ratio.
